# EUROPEAN PATENT SPECIFICATION

(11) **EP 1 506 785 B1**
(45) Date of publication and mention of the grant of the patent: **03.04.2013**
(21) Application number: 03752913.8
(22) Date of filing: 16.05.2003
(51) Int. Cl.: A61K 38/17, A61K 35/12, A61K 35/56, A61K 35/58, A61K 35/60, A61P 3/00, A61P 7/00, A61P 19/06, A23L 1/30

(54) **COMPOSITION FOR TREATING OR PREVENTING HYPERURICEMIA**
ZUSAMMENSETZUNG ZUR BEHANDLUNG ODER PRÄVENTION VON HYPERURIKÄMIE
COMPOSITION POUR TRAITER OU PREVENIR UNE HYPERURICEMIE

(30) Priority: 20.05.2002 JP 2002145285
(43) Date of publication of application: 16.02.2005
(73) Proprietor: Maruha Nichiro Seafoods, Inc., Chiyoda-ku Tokyo (JP)
(72) Inventor: MUROTA, Itsuki, Maruha Corporation, Tsukuba-shi, Ibaraki 300-4295 (JP); NISHIKAWA, Masazumi, Maruha Corporation, Tsukuba-shi, Ibaraki 300-4295 (JP); TAMAI, Tadakazu, Maruha Corporation, Tsukuba-shi, Ibaraki 300-4295 (JP); YOSHIKAI, Kazuyoshi, Maruha Corporation, Tsukuba-shi, Ibaraki 300-4295 (JP); KIHARA, Minoru, Maruha Corporation, Utsunomiya-shi, Tochigi 321-3231 (JP)
(74) Representative: Forstmeyer, Dietmar
(86) International application number: PCT/JP2003/006140
(87) International publication number: WO 2003/097081

(56) References cited:
- WO-A1-00/56275
- WO-A1-93/09766
- WO-A1-98/52583
- JP-A- 6 178 671
- JP-A- 7 033 668
- JP-A- 7 308 169
- JP-A- 2001 231 497

## Description

### Technical Field:

This invention relates to a composition for use in the treatment or prevention of hyperuricemia.

### Background of Art:

Gout is a serious lifestyle-related disease with the recent trend in the improvement of eating habits. Gout is known to be caused by the excess yield of uric acid in the body, which is deposited as calcium urate usually in toes and fingers, resulting in a sudden and severe attack of pain. Options for the treatment of gout include (1) the use of uric acid synthesis inhibitors for inhibiting accumulation of uric acid in the body and (2) the use of uric acid-excretory accelerator or urine alkalizers for accelerating rapid excretion of uric acid accumulated in the body.

Allopurinol is currently easily available as uric acid synthesis inhibitors. Probenecid, sulfinpyrazone, and benzbromarone are known as uricosuric agents. However, allopurinol is known to induce side effects such as systemic hypersensitive symptoms including a skin disease, poisoning syndrome, myelosuppression, and hepatopathy, and therefore needs due considerations in use. The uricosuric agents such as benzbromarone also have problems such that they can precipitate a gout attack if misused.

Chondroitin sulfate is a typical sulfated mucopolysaccharide. There are subtypes A to K, classified by the position and the number of an O-sulfate group(s). It has been reported that chondroitin sulfate A and C exist in cartilage and chondroitin sulfate B in the skin. Chondroitin sulfate subtypes with high sulfate group contents are also found; for example, chondroitin sulfate D in shark cartilage, chondroitin sulfate E in sagittate calamary cartilage, and chondroitin sulfate K in king crab (see Seikagaku Jiten, 3rd ed., p556,Tokyo Kagaku Dojin Co., Ltd., s.v. "Kondoroichin Ryusan").

Chondroitin sulfate is not present freely in normal living body but is present as protein-mucopolysaccharide complexes bound to proteins (proteoglycans) to exhibit its functions (see New Food Industry, vol. 41, No. 9, 1999). Proteoglycans mainly are distributed in cartilage, skin, tendon, ligament, bone, etc. and are particularly abundant in cartilage (see New Food Industry, vol. 40, No. 2, 1998).

The physiology is to constitute polymer matrices for maintaining parenchymal cells and to build up organ-specific structures (see New Food Industry, vol. 31, No. 9, 1999). In addition, it has extremely high water holding capacity and takes part in nutrition transportation and absorption through the water of living body (see New Food Industry, vol. 31, No. 9, 1989). As a result of development of recent biochemical studies and from the experiences in clinical trials including animal experiments, it has been revealed to have ten functions as typical characteristics: body water control, enhancement of osteogenesis, lubrication of joints, blood lipid lowering, control of vascularization, control of substances moving in and out of cells, rapid healing of wounds, infection prevention of bacteria, suppression of blood coagulation, and maintenance of eyes' transparency (see New Food Industry, vol. 41, No. 9, 1999).

For instance, the present applicant reported experiments in which rats with induced osteoporosis were fed on a diet containing 20 to 80 parts by weight of a chondroitin sulfate protein complex per 3 parts by weight of calcium or, in terms of calcium, of a calcium-containing substance or a diet containing no chondroitin sulfate protein complex. According to the results, the group fed on the chondroitin sulfate protein complex-rich diet showed accelerated calcium absorption to gain in bone strength compared with the group fed without the chondroitin sulfate protein complex (see Japanese Patent 3248170).

WO 98/52583 discloses a composition for treating mammals having conditions characterized by bone or joint inflammation, the composition being formed by preparing a mixture comprising: an effective amount of systemically absorbable cartilage, and an effective amount of an aminosaccharide.

WO 93/09766 discloses a method for the treatment of pain in mammals including humans, comprising administering to said mammal in need of such treatment an analgesic effective amount of a sodium chondroitin sulfate selected from sodium chondroitin sulfate C, sodium chondroitin sulfate D and mixtures thereof, said effective amount exhibiting collagenase inhibitory activity and angiogenesis inhibitory activity; wherein said sodium chondroitin sulfate is derived from shark fin cartilage, is essentially free of endotoxin and has a molecular weight in the range of about 18,000 to about 100,000 Daltons.

WO 00/56275 discloses a preparation for use in the treatment or prevention of rheumatic diseases comprising: a) 1 to 50 wt%, relative to the preparation total weight, of at least a compound selected among chondroitin sulfates and their salts; and b) 1 to 66 wt%, relative to the preparation total weight, of at least one compound selected among chitosan, its salts, its derivatives and the salts of said derivatives. In particular, the preparation is used for preventing or treating joint tissue alteration.

### Disclosure of the Invention:

An object of the present invention is to provide a new type of a composition for use in treating or preventing hyperuricemia that has serum uric acid-lowering activity.

The present inventors have conducted extensive investigations to achieve the above object and found as a result that a chondroitin sulfate protein complex lowers a serum uric acid level.

Based on the above finding, the present invention provides a composition for use in treating or preventing hyperuricemia containing a chondroitin sulfate protein complex as an active ingredient.

The present invention also provides a chondroitin sulfate protein complex-containing food characterized by containing the above-described hyperuricemia treating or preventing composition in an amount of 10 mg to 5 g in terms of the chondroitin sulfate protein complex.

### Brief Description of the Drawings:

Fig. 1 is a graph showing changes of serum uric acid levels of the hyperuricemia-inducing diet group and the hyperuricemia-inducing diet + SCP diet group in Experimental Example 2 given infra.
Fig. 2 is a graph showing changes of serum uric acid levels of the hyperuricemia-inducing diet group and the group maintained on the hyperuricemia-inducing diet until the 29th day, on which the diet was switched over to the hyperuricemia-inducing diet + SCP in Experimental Example 2 given infra.
Fig. 3 is a graph comparing serum uric acid levels of the groups after being fed on test diets for 28 days in Experimental Example 3 given infra.
Fig. 4 is a flow chart illustrating the steps for preparing a chondroitin sulfate protein complex.

### Best Mode for Carrying out the Invention:

Preferred embodiments of the composition for use in treating or preventing hyperuricemia according to the present invention will be described in detail.

Being of natural origin and used as an ingredient of commonly eaten foods, the chondroitin sulfate protein complex, the effective ingredient of the composition, has no safety problem. The chondroitin sulfate protein complex includes those derived from cartilaginous fishes, bony fishes, mammals, birds, reptiles, amphibians, crustaceans, and mollusks, with those from cartilaginous fishes being preferred.

The chondroitin sulfate protein complex that can be used in the present invention is prepared in accordance with the flow chart shown in Fig. 4. When starting from cartilage, the cartilage of shark, bovine, etc. is degraded with protease. The digestate is purified (filtered and treated with activated carbon) and, if needed, spray dried. Otherwise, the cartilage is extracted with hot water or decomposed with an acid, e.g., hydrochloric acid, and the extract or decomposition product is purified (filtered and treated with activated carbon) and, if desired, spray dried. Such chondroitin sulfate protein complexes are commercially available under the trade names of SCP or SCP(NB) from Maruha Corp., Kondroitin Q (20%) from Q.P. Corp., SNC from Nichiro Corp, Kondroitin Ryusan (for food) from Shin Nippon Yakugyo Co., Ltd., Kondroitin Ryusan from Yantai Dongchen Biochemicals Co., Ltd., PCS-20 from Pacific Corp., etc. These commercial products can be utilized in the invention.

It is preferred for the chondroitin sulfate protein complex to be used in the invention to have a mucopolysaccharide content of 15% by mass or more, still preferably 30% by mass or more, as measured according to the barium sulfate gravimetric method, or of 6% by mass or more, still preferably 13% by mass or more, as measured according to the carbazole-sulfuric acid method, which is the method for determining mucopolysaccharide/protein contents specified in the mucopolysaccharide/protein food standards set by Japan Health & Nutrition Food Association. It is also preferred for the chondroitin sulfate protein complex to be used in the invention to have a protein content of 4% by mass or more, still preferably 20% by mass or more, as measured according to the semi-micro Kjeldahl method, which is the method for determining mucopolysaccharide/protein contents specified in the mucopolysaccharide/protein food standards set by Japan Health & Nutrition Food Association. It is also preferred for the chondroitin sulfate protein complex to be used in the invention to have a pH of 4.0 to 8.0, more preferably 5.5 to 7.0; a loss on drying of 20.0% by mass or less, more preferably 10.0% by mass or less; an ignition residue (ash content) of 1.0% to 20.0% by mass, more preferably 5.0% to 20.0% by mass, and a lipid content of 5.0% by mass or less, more preferably 1.0% by mass or less.

If desired, the composition of the present invention can contain in addition to the chondroitin sulfate protein complex isotonic agents such as saccharides and salts, buffering agents for adjusting to match in vivo pH, surface active agents, antioxidants, absorption retarding agents, and so forth.

The composition of the invention can be administered orally in dosage forms, such as tablets, powders, granules, lozenges, and capsules, which are formulated by using appropriate solid carriers, such as lactose, clay, sucrose, dextrin, talc, gelatin, agar, pectin, acacia, and magnesium stearate; or syrups, soft gelatin capsules, and drinks, which are formulated by using liquid carriers, such as syrups, vegetable oils, and water.

The composition of the present invention can also be taken as an additive to foods. It can be added to a food in any form in conformity to the food without undergoing impairment of its effects. Hence, the composition is applicable to a wide variety of foods, including bread, French bread, butter rolls, milk shakes, corn soups, potage soups, curries, stews, and the like.

The chondroitin sulfate protein complex content in the composition is decided from the effective dose described below taking the number of doses per day and the daily dose into consideration.

Where the composition of the invention is added to a food, the amount to be added is decided taking the effective dose described below into consideration.

The effective dose of a chondroitin sulfate protein complex is preferably 10 mg to 5 g, still preferably 150 mg to 1 g, in terms of a pure complex, per day for an adult requiring serum uric acid lowering. The daily dose is administered in a single dose or several divided doses. It should be noted that the above-recited daily dose is presented only as a standard so that the daily intake should be decided depending on the age, body weight, sex, symptoms, and other conditions of a person who needs medication. Generally speaking, the time for a person to require lowering of the serum uric acid level is the time when he or she has a gout attack or when a gout attach is expected, for example immediately before, or during, or immediately after eating or drinking. When a person has a serum uric acid level exceeding 7.0 mg/dl, it appears that some medication is needed to lower the level.

The effects of the present invention will further be illustrated by way of Experimental Examples and Examples.

### EXPERIMENTAL EXAMPLE 1

Seven-week-old SD female rats (n=5, 10 rats in total) were given intragastrically 1 g/kg-b.w. of a chondroitin sulfate protein complex derived from shark (SCP from Maruha Corp.) or distilled water (as a control) for 14 days. After the end of the final administration, a blood sample was taken from the abdominal aorta. Serum was separated from the blood sample and tested for 15 biochemical items: GOT, GPT, total cholesterol, HDL-cholesterol, glucose, total bilirubin, total protein, albumin, urea nitrogen, creatinine, Ca, Na, Cl, alkaline phosphatase (ALP), and uric acid. The test results, which are shown in Table 1 below, reveal that the SCP group showed lower uric acid concentrations than the control group with significant difference (p<0.01).

**TABLE 1**

| | Control | SCP Group | T-test |
|---|---|---|---|
| Total Protein (g/dl) | 4.82 | 5.32 | n.s. |
| HDL Cholesterol (mg/dl) | 39.0 | 34.0 | n.s. |
| Albumin (g/dl) | 3.22 | 3.16 | n.s. |
| Total Bilirubin (mg/dl) | 0.24 | 0.26 | n.s. |
| Glucose (mg/dl) | 122.0 | 117.0 | n.s. |
| Total Cholesterol (mg/dl) | 70.0 | 53.7 | n.s. |
| Urea Nitrogen (mg/dl) | 20.3 | 21.0 | n.s. |
| Creatinine (mg/dl) | 0.46 | 0.40 | n.s. |
| Sodium (mEq/l) | 128.2 | 123.8 | n.s. |
| Chlorine (mEq/l) | 89.8 | 86.4 | n.s. |
| GOT (IU/1/37°C) | 56.6 | 49.6 | n.s. |
| GPT (IU/1/37°C) | 16.3 | 17.3 | n.s. |
| ALP (KAU) | 548.0 | 557.0 | n.s. |
| Calcium (mg/100ml) | 9.76 | 9.38 | n.s. |
| Uric Acid (mg/100ml) | 1.13 | 0.47 | p<0.01 |

| | | | |
|---|---|---|---|
| n.s.: No significant difference | | | |

### EXPERIMENTAL EXAMPLE 2

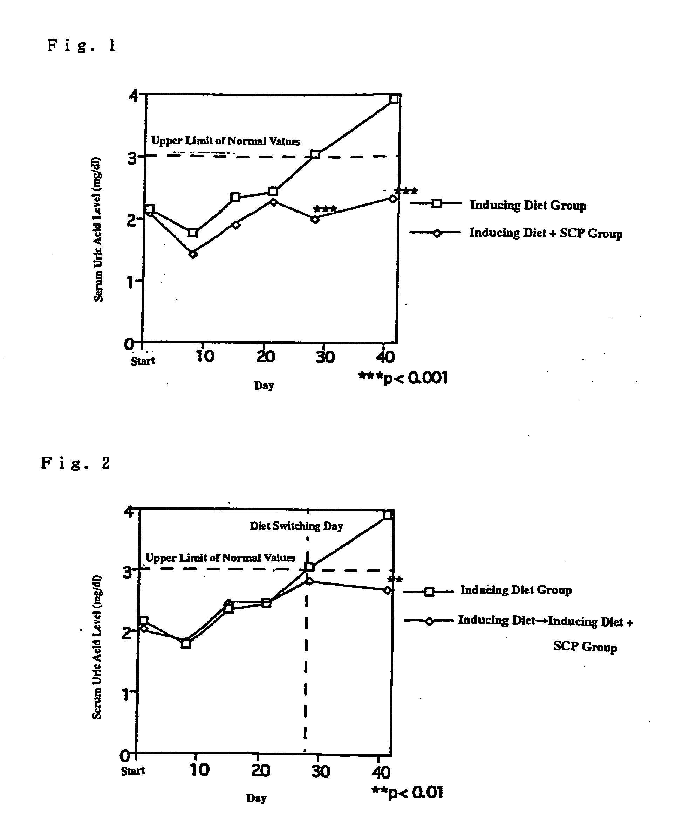

After completion of acclimation and breeding, ten-week-old SD female rats (n=6, 18 rats in total) were fed freely (10 to 15 g/day) on a diet prepared by adding 2.5% by total mass potassium oxonate inducing hyperuricemia to a mixed feed supplied by Oriental Yeast Co., Ltd. (hereinafter referred to as an inducing diet) or a diet prepared by adding 1.5% by total mass of a chondroitin sulfate protein complex derived from shark (SCP: from Maruha Corp.) to the inducing diet (hereinafter referred to as an inducing diet + SCP diet). Changes in serum uric acid level were traced. The test groups were divided into three groups: an inducing diet group, an inducing diet + SCP diet group, and a group in which the inducing diet was switched over to the inducing diet + SCP diet when a difference in serum uric acid level was observed between the inducing diet group and the inducing diet + SCP diet group (hereinafter referred to as an inducing diet→inducing diet + SCP diet group). As a result, the serum uric acid level gradually increased in the inducing diet group but was kept around 2 mg/ml in the inducing diet + SCP diet group. On and after the 29th day from the start of the experiment, the level of the inducing diet + SCP diet group was lower than that of the inducing diet group with significant difference (p<0.001). After switching of diets, the serum uric acid level in the inducing diet→inducing diet + SCP diet group stopped rising. After about 10 days from the day of diet switching, the inducing diet→inducing diet + SCP diet group had a significantly (p<0.01) lower serum uric acid level than the inducing diet group. These results are graphically shown in Figs. 1 and 2.

### EXPERIMENTAL EXAMPLE 3

After completion of acclimation and breeding, four-week-old Wister male rats (n=6, 60 rats in total) were fed on 10 to 17.5 g/day of an inducing diet prepared by adding 3% by total mass of potassium oxonate inducing hyperuricemia to a mixed feed supplied by Oriental Yeast Co., Ltd. or a diet prepared by adding 2.5% by total mass of a chondroitin sulfate protein complex derived from shark, salmon, bovine or chicken (hereinafter referred to as SCP, salmon-derived SCP, bovine-derived SCP, chicken-derived SCP, respectively: all produced by Maruha Corp.) to the inducing diet. Changes in serum uric acid level were traced. As a result, on the 28th day from the start of the experiment, the uric acid levels of the four inducing diet + SCP groups were lower than that of the inducing diet group with significant difference (p<0.05). These results are graphically shown in Fig. 3.

It is judged from the above experimental results that the chondroitin sulfate protein complex has serum uric acid-lowering effect.

The following is Examples of the composition for treating or preventing hyperuricemia according to the present invention and of foods containing the composition.

### EXAMPLE 1

The following [raw materials] was used to manufacture tablets of the inventive composition for treating or preventing hyperuricemia in a usual manner together with common auxiliaries.

### Raw Materials:

| | |
|---|---|
| Chondroitin sulfate protein complex (SCP, from Maruha Corp.) | 150 mg |
| Collagen peptide | 50 mg |
| Extract of grains | 50 mg |
| Vitamin B₂ | 0.06 mg |
| Vitamin C | 5 mg |
| Lactose | 200 mg |
| Cellulose - | 49.89 mg |

The results of analyses on the resulting tablets are shown in Table 2 below.
The analyses were carried out in accordance with the test methods specified in the standards on health supplementary set by Japan Health & Nutrition Food Association. The test methods used in the other analyses described infra also conform to the same standards.

**TABLE 2**

| Items for Analysis | Results | Note |
|---|---|---|
| Riboflavin (vitamin B₂) | 11.3 mg/100 g | - |
| Total ascorbic acid (total vitamin C) | 1.21 g/100 g | 1 |
| Mucopolysaccharide-protein content | 39.5 mass% | 2 |
| Confirmation of Chondoroitin | within the limit | 2 |

| | | |
|---|---|---|
| The values were calculated from the results of the Test Report Nos.: 102032302-001 and 102032302-001-attached (both issued by Japan Food Research Laboratories on March 29, 2002). Note 1: Testing was preceded by conversion into a derivative using a hydrazide. Note 2: The test was carried out in accordance with the method specified in the standards on mucopolysaccharide-protein-containing foods set by Japan Health & Nutrition Food Association. | | |

The results of analyses on the chondroitin sulfate protein complex used in Example 1 (SCP from Maruha Corp.) are shown in Table 3.

**TABLE 3**

| Items of Analysis | Results |
|---|---|
| Mucopolysaccharide content | 24.3 mass% (note 3) |
| Protein content | 40.9 mass% |
| Heavy metal content | ≤20 ppm |
| Arsenic content | ≤2 ppm |
| Standard plate count | ≤(1.0 x 10₃) |
| Coliform group | Negative |

| | |
|---|---|
| Note 3: Determined by the barium sulfate gravimetric method. | |

### EXAMPLE 2

The following [raw materials] was used to manufacture tablets of the inventive composition for treating or preventing hyperuricemia in a usual manner together with common auxiliaries.

### Raw Materials:

| | |
|---|---|
| The same chondroitin sulfate protein complex as used in Example 1 | 150 mg |
| Lactose | 137.9 mg |
| Vitamin C | 0.1 mg |
| Flavoring | 2 mg |
| Stevia | 10 mg |

The results of analyses on the resulting tablets are shown in Table 4 below.

**TABLE 4**

| Items for Analysis | Results | Note |
|---|---|---|
| Mucopolysaccharide-protein content | 41.6 mass% | 4 |
| Confirmation of Chondroitin | within the limit | 4 |
| Chondroitin sulfate/protein ratio | 0.5 | 4 |
| Weight per tablet | 0.298 g | - |

The values were calculated from the results of the Test Report Nos.: 102030514-005 and 102030514-006 (both issued by Japan Food Research Laboratories on March 19, 2002) and the weight per tablet.
Note 4: The test was carried out in accordance with the method specified in the standards on mucopolysaccharide-protein-containing foods set by Japan Health & Nutrition Food Association.

### EXAMPLE 3

Bread was made from the following raw materials in a usual manner.

### Raw Materials:

| | |
|---|---|
| Chondroitin sulfate protein complex | 1 g |
| Strong flour | 248.37 g |
| Dry yeast | 3 g |
| Salt | 5 g |
| Sugar | 10 g |
| Skimmed milk | 3 g |
| Butter | 10 g |

### EXAMPLE 4

Soup was made in a usual manner using the following raw materials. Raw Materials:

| | |
|---|---|
| Chondroitin sulfate protein complex | 1 g |
| Sweet corn | 250.0 g |
| Butter | 30 g |
| Onion | 20 g |
| Wheat flour | 15 g |
| Milk | 200 ml |
| Salt | 1.5 g |
| Pepper | 0.1 g |
| Sugar | 1 g |
| Garlic | 5 mg |
| Celery | 5 mg |
| Laurel leaf | 1 mg |
| Sodium glutamate | 0.1 g |
| Sodium inosinate | 0.01 g |

### EXAMPLE 5

Retort pouch-packed curry was made in a usual manner using the following raw materials.

### Raw Material:

| | |
|---|---|
| Chondroitin sulfate protein complex - | 12.5 g |
| Carrot | 100 g |
| Potato | 300 g |
| Onion | 200 g |
| Beef | 300 g |
| Curry powder mix | 5 g |
| Wheat flour | 30 g |
| Salt | 5 g |
| Pepper | 1 g |
| Sugar | 0.1 g |
| Sodium glutamate | 1 g |

### Industrial Applicability:

The advantage of the present invention consists in providing a new composition for use in treating or preventing hyperuricemia that has serum uric acid lowering action.

## Claims

1. A composition for use in treating or preventing hyperuricemia, the composition containing a chondroitin sulfate protein complex as an effective ingredient.

2. The composition for use as in claim 1, wherein the chondroitin sulfate protein complex is derived from cartilaginous fishes.

3. The composition for use as in claim 1, wherein the chondroitin sulfate protein complex is derived from bony fishes.

4. The composition for use as in claim 1, wherein the chondroitin sulfate protein complex is derived from the mammals or birds.

5. The composition for use as in claim 1, wherein the chondroitin sulfate protein complex is derived from reptiles, amphibians, crustaceans or mollusks.

6. The composition for use as in any one of claims 1 to 5, wherein the chondroitin sulfate protein complex is obtained by purifying protease degradations of cartilage of animals.

7. The composition for use as in any one of claims 1 to 5, wherein the chondroitin sulfate protein complex is obtained by purifying a hot water extract or an acid decomposition product of cartilage of animals.

8. The composition for use as in claim 1, which is formulated into a dosage form providing 10 mg to 5 g of the chondroitin sulfate protein complex per day.

9. The composition for use as in claim 1, wherein the composition is an additive to foods.

## Patentansprüche

1. Zusammensetzung zur Verwendung bei der Behandlung oder der Prävention von Hyperurikämie, wobei die Zusammensetzung einen Chondroitinsulfat- Proteinkomplex als einen wirksamen Bestandteil enthält.

2. Die Zusammensetzung zur Verwendung wie in Anspruch 1, wobei der Chondroitinsulfat- Proteinkomplex aus Knorpelfischen stammt.

3. Die Zusammensetzung zur Verwendung wie in Anspruch 1, wobei der Chondroitinsulfat- Proteinkomplex aus Knochenfischen stammt.

4. Die Zusammensetzung zur Verwendung wie in Anspruch 1, wobei der Chondroitinsulfat- Proteinkomplex aus Säugetieren oder Vögeln stammt.

5. Die Zusammensetzung zur Verwendung wie in Anspruch 1, wobei der Chondroitinsulfat- Proteinkomplex aus Reptilien, Amphibien, Krustentieren oder Mollusken stammt.

6. Die Zusammensetzung zur Verwendung wie in einem der Ansprüche 1 bis 5, wobei der Chondroitinsulfat- Proteinkomplex durch Reinigen der Protease- Abbauprodukte von Knorpel aus Tieren erhalten wird.

7. Die Zusammensetzung zur Verwendung wie in einem der Ansprüche 1 bis 5, wobei der Chondroitinsulfat- Proteinkomplex durch Reinigen eines Heißwasserextrakts von Knorpel aus Tieren oder eines Zersetzungsprodukts von Knorpel aus Tieren mit Säure erhalten wird.

8. Die Zusammensetzung zur Verwendung wie in Anspruch 1, die als eine Dosierungsform formuliert ist, welche 10 mg bis 5 g des Chondroitinsulfat- Proteinkomplexes pro Tag bereitstellt.

9. Die Zusammensetzung zur Verwendung wie in Anspruch 1, wobei die Zusammensetzung ein Zusatz für Lebensmittel ist.

## Revendications

1. Composition à utiliser dans le traitement ou la prévention de l'hyperuricémie, la composition contenant un complexe protéinique de chondroïtine sulfate en tant qu'un principe actif.

2. Composition à utiliser selon la revendication 1, dans laquelle le complexe protéinique de chondroïtine sulfate est dérivé de poissous cartilagineux.

3. Composition à utiliser selon la revendication 1, dans laquelle le complexe protéinique de chondroïtine sulfate est dérivé de poissons osseux.

4. Composition à utiliser selon la revendication 1, dans laquelle le complexe protéinique de chondroïtine sulfate est dérivé des mammifères ou des oiseaux.

5. Composition à utiliser selon la revendication 1, dans laquelle le complexe protéinique de chondroïtine sulfate est dérivé de reptiles, d'amphibiens, de crustacés ou de mollusques.

6. Composition à utiliser selon l'une quelconque des revendications 1 à 5, dans laquelle le complexe protéinique de chondroïtine sulfate est obtenu par purification de cartilage d'animaux dégradé par la protéase.

7. Composition à utiliser selon l'une quelconque des revendications 1 à 5, dans laquelle le complexe protéinique de chondroïtine sulfate est obtenu par purification d'un extrait à l'eau chaude de cartilage d'animaux ou d'un produit de la décomposition de cartilage d'animaux par acide.

8. Composition à utiliser selon la revendication 1, qui est préparée sous une forme galénique fournissant 10 mg à 5 g du complexe protéinique de chondroïtine sulfate par jour.

9. Composition à utiliser selon la revendication 1, dans laquelle la composition est un additif pour des produits alimentaires.
